# FASCICULE DE BREVET EUROPEEN

(11) **EP 0 996 063 B1**
(45) Date de publication et mention de la délivrance du brevet: **21.05.2003**
(21) Numéro de dépôt: 99402581.5
(22) Date de dépôt: 19.10.1999
(51) Int. Cl.: G06F 11/18, G06F 19/00

(54) **Dispositif médical implantable actif, comprenant des registres protégés pour l'ajustement numérique de paramètres de fonctionnement**
Aktive implantierbare medizinische Vorrichtung mit geschützten Registern zur digitalen Anpassung von Arbeitsparametern
Active implantable medical device comprising protected registers for the digital adjustment of working parameters

(30) Priorité: 19.10.1998 FR 9813056
(43) Date de publication de la demande: 26.04.2000
(73) Titulaire: ELA MEDICAL, F-92541 Montrouge (FR)
(72) Inventeur: Legay, Thierry, 91640 Fontenay les Briis (FR)
(74) Mandataire: Dupuis-Latour, Dominique

(56) Documents cités:
- EP-A- 0 791 373
- WO-A-87/07793
- DE-A- 3 312 873
- US-A- 5 031 180
- US-A- 5 792 201

## Description

La présente invention concerne de façon générale les "dispositifs médicaux actifs". Il peut s'agir de dispositifs implantables tels que définis par la directive 90/385/CEE du 20 juin 1990 du Conseil des communautés européennes - stimulateurs cardiaques, défibrillateurs et/ou cardioverteurs, appareils neurologiques, pompes de diffusion de substances médicales, implants cochléaires, etc. - ou non implantables, c'est-à-dire portés par le patient - notamment les enregistreurs Holter, c'est-à-dire les appareils permettant d'effectuer en continu et sur une longue période l'enregistrement de signaux recueillis au moyen d'électrodes implantées ou d'électrodes externes.

Dans ces dispositifs, comme dans de très nombreux autres dispositifs électroniques, il est nécessaire de régler et de mémoriser un certain nombre de paramètres de fonctionnement.

Parmi ces paramètres, on trouve tout d'abord des paramètres de nature technique, généralement ajustés en production au moment de la calibration de l'appareil (références de tension ou de courant, fréquence d'un oscillateur, gain d'amplificateurs, etc.). En effet, la conception des circuits du dispositif nécessite souvent que le fonctionnement des circuits matériels soit ajusté afin d'obtenir un comportement optimal. Ces ajustages peuvent être réalisés de diverses façons, par exemple par ajustement de résistances par sablage ou gravure laser ; plus récemment, la généralisation des électroniques à capacités commutées et des traitements numériques a ramené le problème de l'ajustage à l'obtention d'un code numérique sur N bits qui, une fois obtenu, est utilisé via des convertisseurs numérique/analogique ou bien pour commander des commutateurs logiques d'une échelle de capacités, ou encore comme variable dans un logiciel. Une telle technique est par exemple exposée dans le EP-A-0 661 657 (ELA Médical).

D'autres paramètres sont ajustés ultérieurement.

Dans le cas particulier d'un dispositif médical actif implantable, certains paramètres peuvent ainsi être ajustés après implantation par le praticien, par exemple pour adapter la prothèse au patient d'un point de vue physiologique. Ces paramètres conditionneront le comportement des divers algorithmes du logiciel de commande du dispositif médical, par exemple pour la délivrance d'impulsions de stimulation ou de chocs de défibrillation dans le cas d'une prothèse cardiaque.

Ces divers paramètres ont comme caractéristique commune d'être des paramètres permanents (c'est-à-dire ajustés une fois pour toutes) ou quasi-permanents, et d'être définis sous forme de codes numériques sur N bits (le nombre N de bits pouvant varier en fonction du paramètre considéré).

Ces codes numériques sont donc stockés dans des mémoires permanentes, qui peuvent être de type ROM à fusibles, EPROM, E²PROM, flash ou encore RAM sauvegardées. Chacun de ces types de mémoires possède ses avantages et inconvénients propres, et surtout une aptitude plus ou moins grande à conserver l'information de façon durable. La RAM, qui possède la plus grande facilité d'écriture, présente aussi un risque plus grand de perte ou de corruption de l'information sur des événements rares, comme par exemple les impacts d'ions lourds tels que les particules alpha ou les parasites électriques susceptibles d'être produits par exemple à la suite d'une exposition à un champ électromagnétique impulsionnel de forte intensité.

Les mémoires de type ROM, EPROM, E²PROM ou flash sont, par nature, bien protégées contre ces risques d'altération, mais elles présentent, pour un dispositif médical actif, un double inconvénient :
- d'une part, du point de vue de la topologie des circuits, ces mémoires ne sont pas physiquement localisées là ou la valeur qu'elles mémorisent est utilisée, et par ailleurs sont organisées architecturalement d'une façon globale en plan mémoire qui interdit l'accès individuel, direct et permanent à l'information, alors que la fonction analogique ajustée a besoin de cette information en permanence ; - en second lieu, la lecture de ces mémoires consomme de l'énergie, paramètre critique pour les prothèses implantables et, plus généralement, pour les dispositifs portables alimentés par une pile, qui empêchent un accès permanent à l'information d'étalonnage de la fonction analogique conservée dans ces mémoires, pour des questions évidentes de consommation d'énergie.

Ces difficultés ont conduit à mémoriser les informations en question dans des registres de type RAM ou bascules électroniques logiques, localisés à l'intérieur du module qui les utilise, donc topologiquement proche du lieu de la fonction qu'ils étalonnent, et lisibles en permanence et à très faible consommation.

Ces registres étant par nature des registres volatils, il faut tenir compte du risque d'effacement accidentel de la donnée, avec deux problèmes :
- surveillance de l'information conservée dans les registres afin d'en contrôler l'intégrité, et ceci avec une consommation négligeable et de façon immédiate ou quasi-immédiate ; et
- correction, également immédiate ou quasi-immédiate, de la donnée corrompue en cas d'altération accidentelle.

Il a été proposé divers systèmes basés sur un codage redondant et/ou auto-correcteur de l'information, par exemple de type CRC ou analogue, voir par exemple le document US-A-5 792 201.

Mais cette technique présente le double inconvénient de nécessiter une scrutation ou analyse quasi-permanente de l'information, impliquant donc une consommation d'énergie importante, et d'autre part de l'apparition d'un état indéterminé de l'information pendant le laps de temps séparant la détection de l'erreur de sa correction, inconvénient qui n'est pas admissible dans le cas de dispositifs médicaux actifs tels que des stimulateurs cardiaques, dans lesquels la continuité d'un fonctionnement correct doit être assurée en toutes circonstances.

L'invention apporte une solution à ces problèmes, en proposant un dispositif médical implantable actif comprenant au moins un module pour la mise en oeuvre d'une fonction du dispositif, ce module comportant un circuit spécifique à la fonction et dont un paramètre est ajustable sous forme d'un mot numérique de N bits, dispositif caractérisé en ce que chacun desdits modules comprend : au moins trois registres de type volatil de N bits mémorisant chacun ledit mot numérique ; un circuit majoritaire recevant en entrée les contenus des registres et délivrant en sortie au circuit spécifique la valeur résultante comme paramètre d'ajustement; et des moyens comparateurs, pour comparer les contenus respectifs des registres et produire un signal d'anomalie en cas de discordance entre ces contenus.

Lorsque le dispositif médical actif est un dispositif à microcontrôleur, le signal d'anomalie peut être un signal d'interruption appliqué au microcontrôleur ; en cas de pluralité de modules, les signaux d'interruption respectifs sont appliqués au microcontrôleur via des moyens pour multiplexer, coder et gérer les priorités de ces interruptions.

Très avantageusement, il est prévu en outre des moyens de restauration du contenu du ou des registre(s) altéré(s) en cas de discordance détectée par les moyens comparateurs. Ces moyens peuvent notamment être des moyens pour rechercher une valeur concordante entre une pluralité de registres et recopier cette valeur dans le registre altéré, ou encore, lorsque la valeur du paramètre est également conservée dans une mémoire non volatile extérieure au module, des moyens pour recopier dans le registre altéré, ou bien dans tous les registres, la valeur lue dans cette mémoire non volatile extérieure.

On va maintenant décrire un exemple de mise en oeuvre de l'invention, en référence aux dessins annexés.

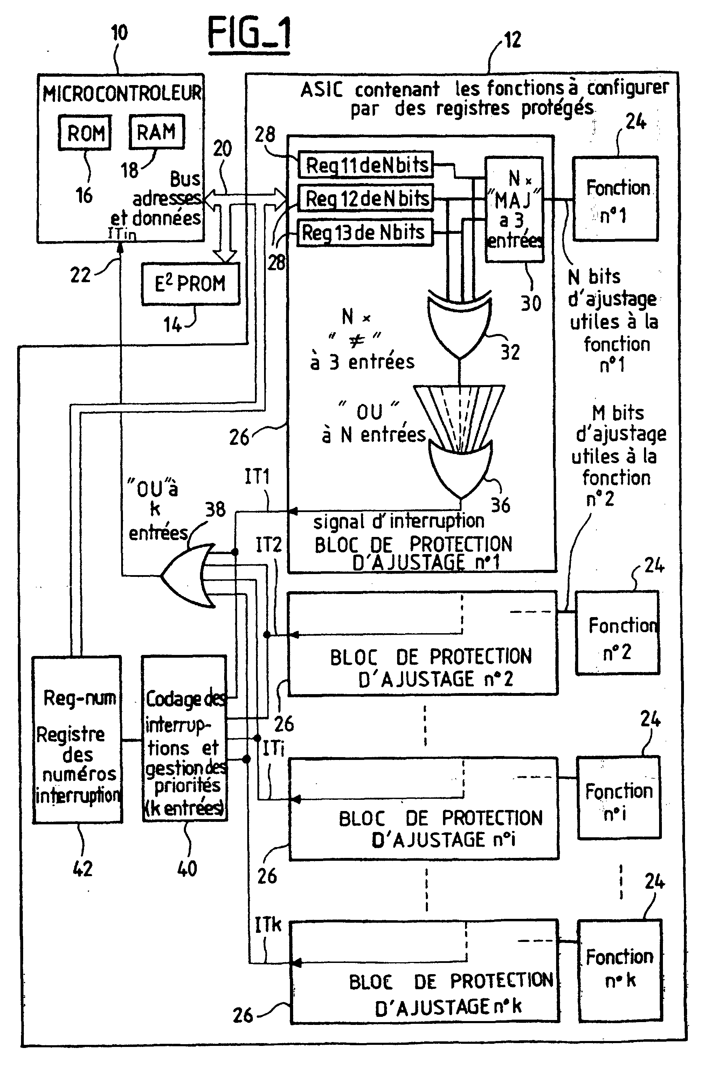

La figure 1 représente, sous forme de schéma par blocs, les principaux éléments d'un dispositif médical actif incorporant le circuit de surveillance et de correction selon l'invention.
La figure 2 montre plus en détail la structure de l'un des blocs référencés 26 de la figure 1.

Le dispositif médical actif se compose essentiellement d'un microcontrôleur 10 et d'un ASIC 12 contenant les diverses fonctions à configurer par des registres protégés.

Il peut éventuellement comporter une mémoire de stockage non volatile 14 telle qu'une E²PROM, qui peut être externe, comme illustré, ou bien interne au microcontrôleur 10.

Le microcontrôleur 10 comporte, de manière classique, une mémoire morte ROM 16 et une mémoire vive RAM 18, et il communique avec l'ASIC 12 et, le cas échéant, l'E²PROM 14 par un bus d'adresses et de données 20.

Il dispose par ailleurs d'une entrée ITᵢₙ 22 dont le rôle sera expliqué plus bas.

L'ASIC 12 comporte un certain nombre de modules 24 dont chacun correspond à une fonction particulière dont un paramètre est ajustable, par exemple :
- amplificateur à gain ajusté,
- oscillateur à fréquence ajustée,
- chaîne de traitement de l'accélération, dans le cas d'un dispositif asservi,
- étage de stimulation à configuration programmable (configuration des électrodes pour un stimulateur multisite, configuration des chocs à appliquer pour un défibrillateur).

On peut également stocker de la même façon des données critiques pour le fonctionnement du dispositif, par exemple le numéro de modèle, qui est une donnée indispensable notamment à la reconnaissance du dispositif par un programmateur externe.

Ces divers modules 24 sont chacun reliés à un bloc 26 mémorisant les N bits (N étant un nombre variable selon la fonction considérée) nécessaires à l'ajustage du module 24.

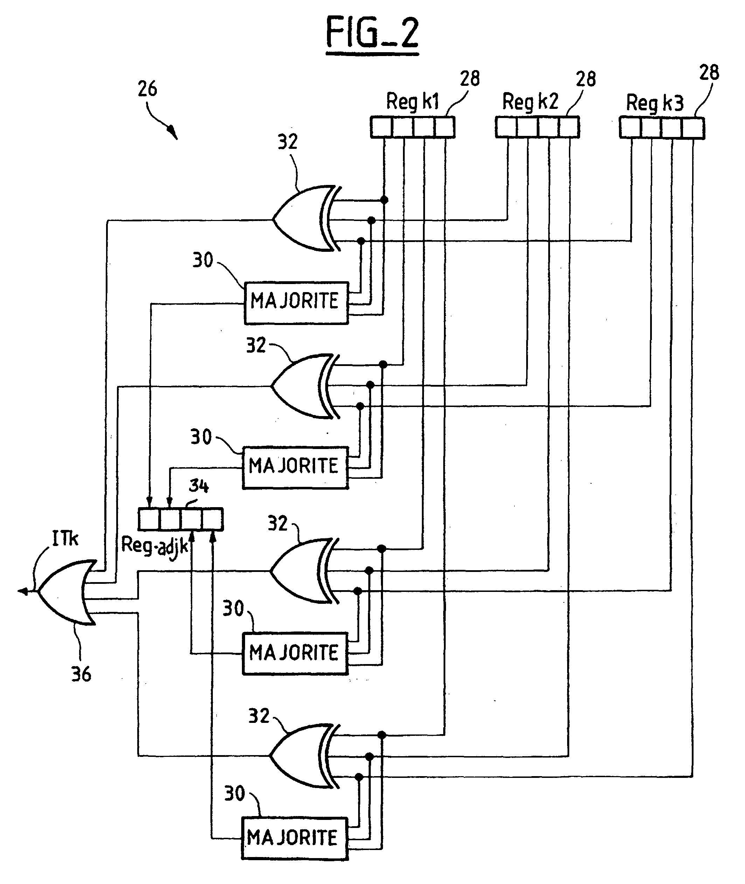

Les bits d'ajustage, organisés en mots de N bits, sont stockés de façon triple dans des registres identiques 28, dont l'architecture est identique d'un bloc 26 à l'autre, mais qui diffèrent en taille suivant le nombre N de bits d'ajustage correspondant au module 24 associé (la figure 2 illustre ainsi un exemple de bloc 26 dans le cas où N = 4).

Le chiffre de trois pour le nombre de registres 28 n'est pas limitatif, et un nombre supérieur peut être envisagé, de préférence (mais non nécessairement) un nombre impair.

Initialement, et dans les conditions normales de fonctionnement, les trois registres 28 d'un même bloc 26 stockent la même information.

Chacun des bits de chacun des trois registres 28 d'un même bloc 26 est appliqué, d'une part, à l'une des trois entrées d'un circuit majoritaire respectif 30 et, d'autre part, à l'une des trois entrées d'une porte respective 32 réalisant la fonction DIFFÉRENCE (sortie à 'faux' si et seulement si les trois entrées sont identiques (toutes à '0' ou toutes à '1'), et sortie à 'vrai' dans le cas contraire).

Pour mémoire, la table de vérité d'un circuit majoritaire à trois entrées A, B et C est la suivante :

| A | B | C | Majorité |
|---|---|---|---|
| 0 | 0 | 0 | 0 |
| 0 | 0 | 1 | 0 |
| 0 | 1 | 0 | 0 |
| 0 | 1 | 1 | 1 |
| 1 | 0 | 0 | 0 |
| 1 | 0 | 1 | 1 |
| 1 | 1 | 0 | 1 |
| 1 | 1 | 1 | 1 |

La table de vérité d'un circuit DIFFÉRENCE à trois entrées A, B et C, quant à elle, est la suivante :

| A | B | C | Différence |
|---|---|---|---|
| 0 | 0 | 0 | 0 |
| 0 | 0 | 1 | 1 |
| 0 | 1 | 0 | 1 |
| 0 | 1 | 1 | 1 |
| 1 | 0 | 0 | 1 |
| 1 | 0 | 1 | 1 |
| 1 | 1 | 0 | 1 |
| 1 | 1 | 1 | 0 |

Les sorties des circuits majoritaires 30 sont appliqués à un registre Reg_Adj 34 permettant l'ajustage de la fonction du module 24 associé au bloc 26 considéré. Ainsi, si une altération survient sur l'un des trois registres 28, aucune conséquence n'est vue par la fonction ajustée.

Ces circuits majoritaires garantissent donc l'intégrité du système même si l'un des registres vient à être altéré, car dans ce cas les deux autres registres resteront identiques et c'est leur valeur qui sera prise en compte par le circuit majoritaire 30, de sorte qu'aucune conséquence de l'altération survenue ne soit vue par la fonction ajustée.

Les portes DIFFÉRENCE 32, en comparant bit à bit les trois registres 28, surveillent que ces trois registres 28 sont toujours identiques. Si tel est le cas (situation normale) les portes 32 sont à l'état inactif (la consommation est donc nulle, bien que la surveillance soit permanente) ; dans le cas où une différence apparaît entre les trois registres sur l'un quelconque des bits, la fonction de la porte DIFFÉRENCE 32 correspondante passe à l'état actif, révélant ainsi l'altération de l'un des registres.

Le circuit va gérer la détection d'une telle altération de la manière suivante.

Les sorties des portes DIFFÉRENCE 32 de chaque bloc 26 sont reliées à l'une des entrées d'une porte OU commune 36 à N entrées (autant d'entrées que de bits dans chacun des registres 28) produisant en sortie un signal, permettant de véhiculer une demande d'interruption en direction du microcontrôleur.

Ainsi, pour chaque fonction, un seul fil est nécessaire pour véhiculer la demande d'interruption propre à l'ajustage du module correspondant, quel que soit le bit altéré dans les trois registres 28.

L'ensemble des demandes d'interruption IT₁, IT₂, ... est regroupé et traité par deux fonctions :
- une porte OU 38 à k entrées regroupe toutes les demandes d'interruption, et sa sortie est interfacée avec l'entrée d'interruption 22 du microcontrôleur 10 ;
- un bloc 40 à k entrées a pour fonction de calculer le numéro de l'interruption qui s'est produite et l'écrire dans un registre Reg_Num 42 accessible en lecture dans le système global via le bus 20; si plusieurs interruptions se produisent en même temps, le bloc 40 mémorise chacune des demandes et inscrit dans le registre 42 le numéro de l'interruption à traiter, en fonction de priorités prédéfinies.

Le microcontrôleur 10, sur réception d'une demande d'interruption ITᵢₙ 22, exécute alors un programme spécifique qui peut être contenu dans la ROM 16 ou la RAM 18 pour traiter le problème de corruption de registre qui vient de se produire. Pour savoir quel ajustage vient d'être altéré, il lui suffit de lire le registre Reg_Num 42 pour obtenir son numéro et donc l'adresse du (des) registre(s) 28 altéré(s).

Deux modes opératoires peuvent être prévus, suivant que le système possède ou non une unité de stockage non volatile 14 dans laquelle se trouvent également mémorisées les valeurs des paramètres d'ajustage.

Dans un premier cas, en l'absence d'unité de stockage non volatile, c'est-à-dire si les valeurs des paramètres sont exclusivement conservées dans les registres 28, le microcontrôleur lit les trois registres 28 correspondant au numéro d'interruption indiqué par le registre 42, et vérifie qu'au moins deux des registres 28 sur trois sont identiques. Il réécrit dans ce cas le troisième registre identique aux deux autres ; si les trois registres sont différents, une valeur de compromis est inscrite dans les trois registres.

Si, en revanche, le paramètre d'ajustement est inscrit dans une mémoire non volatile telle que 14, alors le microcontrôleur lit la valeur de référence de l'ajustage signalée par son numéro d'interruption dans cette mémoire non volatile et réécrit cette valeur dans les trois registres 28 concernés (on prévoit dans ce cas une interruption masquable).

En variante, au lieu de corriger spécifiquement celui des registres qui a été altéré, on peut réécrire l'ensemble des registres 28 chaque fois qu'une altération est détectée. Cette manière de procéder évite le recours aux circuits de codage des interruptions et de gestion des priorités 40 et 42 ; il est cependant nécessaire, dans ce cas, de prévoir une interruption masquable pendant toute la durée de la correction.

On notera incidemment que, si l'on conserve en mémoire non volatile les paramètres d'ajustage, le système peut gérer non seulement la corruption des données par des phénomènes parasites, mais également les pertes transitoires d'alimentation.

Lorsque le microcontrôleur a achevé l'étape de correction, il rétablit la surveillance du système en autorisant de nouveau la ligne d'interruption 22 affectée à la protection des ajustages. Si plusieurs demandes avaient été enregistrées simultanément, après que le microcontrôleur ait traité la demande prioritaire, dès la réautorisation le système de protection génère à nouveau une demande avec un numéro différent dans le registre Reg_Num 42.

Comme on l'aura compris, cette correction, gérée par une simple interruption (prioritaire) du microprocesseur, est très rapidement opérée, typiquement en quelques millisecondes, temps très court permettant de ne pas affecter le comportement physiologique du dispositif médical actif.

On notera par ailleurs que l'intégrité du système est garantie même pendant la phase (très courte) de correction par le microprocesseur de la donnée corrompue, grâce au circuit majoritaire 30 qui continue à appliquer au module 24 la valeur correcte, contenue dans les registres non altérés.

## Revendications

1. Un dispositif médical implantable actif, comprenant au moins un module (12) pour la mise en oeuvre d'une fonction du dispositif, ce module comportant un circuit (24) spécifique à la fonction et dont un paramètre est ajustable sous forme d'un mot numérique de N bits,
dispositif **caractérisé en ce que** chacun desdits modules comprend :
- au moins trois registres (28) de type volatil de N bits mémorisant chacun ledit mot numérique,
- un circuit majoritaire (30) recevant en entrée les contenus des registres et délivrant en sortie audit circuit spécifique (24) la valeur résultante comme paramètre d'ajustement, et
- des moyens comparateurs (32, 36), pour comparer les contenus respectifs des registres et produire un signal d'anomalie (IT₁, IT₂ ... ITᵢ ... ITₖ) en cas de discordance entre ces contenus.

2. Le dispositif de la revendication 1, dans lequel le dispositif médical actif est un dispositif à microcontrôleur (10), et le signal d'anomalie est un signal d'interruption appliqué au microcontrôleur.

3. Le dispositif de la revendication 2, comportant une pluralité desdits modules, et dans lequel les signaux d'interruption respectifs sont appliqués au microcontrôleur via des moyens (38, 40, 42) pour multiplexer, coder et gérer les priorités de ces interruptions.

4. Le dispositif de la revendication 1, comportant en outre des moyens de restauration du contenu du ou des registre(s) altéré(s) en cas de discordance détectée par les moyens comparateurs.

5. Le dispositif des revendications 1 et 4 prises en combinaison, dans lequel les moyens de restauration sont des moyens pour rechercher une valeur concordante entre une pluralité de registres et recopier cette valeur dans le registre altéré.

6. Le dispositif de la revendication 4, dans lequel la valeur du paramètre est également conservée dans une mémoire non volatile (14) extérieure au module (12), et les moyens de restauration sont des moyens pour recopier dans le registre altéré la valeur lue dans cette mémoire non volatile extérieure.

7. Le dispositif de la revendication 6, dans lequel les moyens de restauration recopient dans tous les registres la valeur lue dans la mémoire non volatile extérieure.

## Claims

1. An active medical device, including at least one module (12) for achieving a function of the device, said module comprising a circuit (24) specific to said function and a parameter of which is adjustable in the form of a digital word of N bits, said device being **characterized in that** each of said modules comprises:
- at least three volatile-type, N-bits registers (28), each storing said digital word,
- a majority circuit (30) receiving on its input the contents of the registers and issuing on its output to said specific circuit (24) the resulting value as adjustment parameter, and
- comparator means (32, 36), for comparing the respective contents of the registers and issuing an anomaly signal (IT₁, IT₂ ... ITᵢ ... ITₖ) in the event of a mismatch between said contents.

2. The device of claim 1, wherein the active medical device is a microcontroller (10) device, and the anomaly signal is an interrupt signal applied to the microcontroller.

3. The device of claim 2, comprising a plurality of said modules, and wherein the respective interrupt signals are applied to the microcontroller through means (38, 40, 42) for multiplexing, coding and managing the priorities of said interruptions.

4. The device of claim 1, further comprising means for restoring the contents of the corrupted register(s) in the event of a mismatch detected by the comparator means.

5. The device of claims 1 and 4 taken in combination, wherein the restoring means are means for finding a matching value between a plurality of registers and recopying said value in the corrupted register.

6. The device of claim 4, wherein the value of the parameter is further stored in a non-volatile memory (14) external to the module (12), and the means for restoring are means for recopying in the corrupted register the value read in said external non-volatile memory.

7. The device of claim 6, wherein the means for restoring recopy in all the registers the value read in the non-volatile memory.

## Patentansprüche

1. Aktive medizinische Vorrichtung, aufweisend zumindest ein Modul (12) für das Realisieren einer Funktion der Vorrichtung, wobei dieses Modul eine für die Funktion spezifische Schaltung (24) aufweist und wovon ein Parameter in Form eines numerischen Wortes von N Bits einstellbar ist, wobei die Vorrichtung **dadurch gekennzeichnet ist, dass** jedes der Module aufweist:
- zumindest drei Register (28) des volatilen Typs von N Bits, wovon jedes das numerische Wort speichert,
- eine Hauptschaltung (30), welche am Eingang die Inhalte der Register empfängt und am Ausgang zur spezifischen Schaltung (24) den resultierenden Wert als Einstellparameter liefert, und
- Komparatormittel (32, 36), um die jeweiligen Inhalte der Register zu vergleichen und ein Anomalie-Signal (IT₁, IT₂, ... ITᵢ ... ITₖ) zu erzeugen im Fall einer Ungleichheit zwischen diesen Inhalten.

2. Vorrichtung nach Anspruch 1, bei welcher die aktive medizinische Vorrichtung eine Vorrichtung mit Mikrocontroller (10) ist und das Anomalie-Signal ein Unterbrechungssignal ist, das an den Mikrocontroller angelegt wird.

3. Vorrichtung nach Anspruch 2, welches eine Mehrzahl der Module aufweist, und bei welcher die jeweiligen Unterbrechungssignale an den Mikrocontroller über Mittel (38, 40, 42) angelegt werden, um die Prioritäten dieser Unterbrechungen zu multiplexen, zu codieren und zu verwalten.

4. Vorrichtung nach Anspruch 1, welche darüber hinaus Wiederherstellungsmittel des Inhaltes oder des oder der geänderten Register im Fall einer durch die Komparatormittel detektierten Ungleichheit aufweist.

5. Vorrichtung nach Anspruch 1 und 4 in Kombination, bei welcher die Wiederherstellungsmittel Mittel sind, um einen Konkordanzwert zwischen einer Mehrzahl von Registern zu suchen und um diesen Wert in das geänderte Register zurückzukopieren.

6. Vorrichtung nach Anspruch 4, bei welcher der Wert des Parameters auch in einem nicht volatilen Speicher (14) aufbewahrt wird außerhalb des Moduls (12) und die Wiederherstellungsmittel Mittel sind, um in das geänderte Register den Wert zu kopieren, der in diesem nicht volatilen externen Speicher gelesen wird.

7. Vorrichtung nach Anspruch 6, bei welcher die Wiederherstellungsmittel den Wert in alle Register kopieren, der in dem nicht volatilen externen Speicher gelesen wurde.
